# FASCICULE DE BREVET EUROPEEN

(11) **EP 0 927 014 B1**
(45) Date de publication et mention de la délivrance du brevet: **12.09.2001**
(21) Numéro de dépôt: 97929345.3
(22) Date de dépôt: 13.06.1997
(51) Int. Cl.: A61F 13/08, D04B 9/52

(54) **ORTHESE COMPRESSIVE DE TYPE BOTTE POUR LE TRAITEMENT DES PATHOLOGIES CIRCULATOIRES DES MEMBRES INFERIEURS, NOTAMMENT POUR LA CONTENTION DE LA JAMBE A LA SUITE D'UN ULCERE VEINEUX**
KOMPRESSIONSORTHESE IN SCHUHFORM UM ZIRKULATIONSKRANKHEITEN IN DEN UNTEREN GLIEDMASSEN ZU BEHANDELN UND INSBESONDERE DAS BEIN RUHIG ZU STELLEN NACH VENENGESCHWÜREN
BOOT-SHAPED COMPRESSIVE ORTHOSIS FOR TREATING CIRCULATORY DISEASES IN THE LOWER LIMBS, AND PARTICULARLY FOR IMMOBILISING A LEG FOLLOWING A VENOUS ULCER

(30) Priorité: 14.06.1996 FR 9607397
(43) Date de publication de la demande: 07.07.1999
(73) Titulaire: Innothera Topic International, 94110 Arcueil (FR)
(72) Inventeur: GARDON-MOLLARD, Christian, F-63400 Chamalières (FR)
(74) Mandataire: Dupuis-Latour, Dominique
(86) Numéro de dépôt international: FR9701067
(87) Numéro de publication internationale: WO9747262

(56) Documents cités:
- EP-A- 0 071 818
- DE-A- 2 843 991
- DE-C- 19 503 459
- FR-A- 2 635 001
- GB-A- 1 445 233
- US-A- 3 386 270
- US-A- 3 889 494
- US-A- 4 086 790

## Description

L'invention a trait au domaine des orthèses compressives de type botte pour le traitement des pathologies circulatoires des membres inférieurs.

Dans la présente description, le terme d"'orthèse" sera entendu dans son sens médical classique, à savoir un appareil d'assistance appliqué à une région du corps pour pallier une déficience fonctionnelle du système locomoteur, à l'exclusion des prothèses qui sont des appareils de suppléance. Une "botte" est une orthèse enveloppant la jambe et le pied, étant entendu que cette botte peut remonter, selon les cas, jusqu'au jarret, jusqu'au milieu de la cuisse ou jusqu'au haut de la cuisse et que le pied n'est pas nécessairement enveloppé complètement, au contraire il est souvent préférable d'arrêter la botte par exemple à la racine des orteils.

Des orthèses de ce type sont notamment décrites dans les GB-A-1 445 233, US-A-3 386 270, FR-A-2 635 001, EP-A-0 071 818 et DE-C-195 03 459. Le DE-A-2 843 991, quant à lui, décrit une stucture de maille particulière, sécable, adaptée à une orthèse de type bandage.

Dans le cas présent, la déficience fonctionnelle en cause est une déficience circulatoire d'un membre inférieur, déficience qui sera palliée par application d'une contention, plus précisément d'une contention dégressive, c'est-à-dire d'une compression appliquée sur toute ou partie de la longueur du membre inférieur à partir de la cheville et diminuant au fur et à mesure que l'on s'éloigne de la cheville.

Une des pathologies les plus fréquente, à laquelle s'applique avantageusement l'invention, est le traitement d'un ulcère veineux ou d'une plaie nécessitant une compression. En effet, la guérison des ulcères veineux de jambe passe nécessairement par une contention adaptée et efficace, la compression étant l'élément fondamental du traitement.

Actuellement, dans la très grande majorité des cas cette compression est réalisée par des bottes élasto-compressives inamovibles (BECI), mises en place par le praticien à l'aide de bandes élastiques adhésives qui ne sont utilisées qu'une seule fois ou de bandes sèches lavables, mais qui perdent rapidement leurs qualités compressives initiales. Le pansement est changé tous les trois à dix jours, en fonction de la production d'exsudat de la plaie, la durée moyenne du traitement étant d'environ 70 jours pour un ulcère variqueux non compliqué de surface inférieure à 15 cm².

Les inconvénients de ces BECI sont nombreux :
― nécessité d'une protection de la peau par un jersey ou une couche de mousse,
― nécessité d'un opérateur entraîné pour la mise en place de la bande qui sera laissée en place plusieurs jours,
― imprécision de la pression d'application de la bande, celle-ci dépendant beaucoup de l'habilité de l'opérateur,
― plis gênants de la bande en ambulatoire, et
― mauvaise stabilité verticale des bandages en ambulatoire.

Une compression automatique, dégressive, par bas de contention serait mieux adaptée à un tel traitement et beaucoup plus confortable pour les patients, tout en restant aussi efficace que les BECI sur le plan thérapeutique. Mais actuellement les bas de contention ne sont que très peu utilisés pour cette indication car il s'agit de produits coûteux. Les ulcères de jambes coulant beaucoup, les bas sont souillés et détériorés rapidement, ce qui rend prohibitif le coût du traitement qui est toujours très long, comme on l'a indiqué plus haut.

La présente invention a pour objet de remédier à cette difficulté, en proposant un nouveau type d'orthèse compressive de type botte (au sens indiqué plus haut) du type générique divulguée par le GB-A-1 445 233 précité, qui remplisse l'ensemble des conditions suivantes :
― produit à usage unique,
― produit de faible coût,
― produit facile à manipuler pour le praticien,
― produit à taille unique, ajustable en fonction de la longueur de la jambe,
― contention efficace pour la pathologie particulière envisagée,
― contention dégressive,
― compression sur le pied pratiquement molle,
― réajustement possible sans retrait du produit,
― compatibilité avec un port continu du produit, pendant plusieurs jours voire plusieurs semaines, ainsi qu'en décubitus (typiquement, la nuit).

À cet effet, l'invention propose une orthèse selon la revendication 1. Les sous-revendications visent des caractéristiques subsidiaires avantageuses.

D'autres caractéristiques et avantages de l'invention apparaîtront à la lecture de la description détaillée ci-dessous.

La figure 1 est une vue en élévation, côté postérieur, de la botte de l'invention à l'état libre, avant mise en place.

La figure 2 est une vue en élévation, côté latéral, de la botte de l'invention, enfilée sur la jambe et le pied d'un patient.

La figure 3 est une vue du tube tricoté en continu, selon une première mise en oeuvre de l'invention.

La figure 4 illustre une seconde forme de mise en oeuvre de l'invention.

La figure 5 illustre une première structure de maille possible pour le tricotage du tube.

La figure 6 illustre une seconde structure de maille possible pour ce tricotage.

Les figures 7 et 8 représentent deux variantes possibles pour la séparation du tube en bottes individuelles.

La figure 9 illustre la texture du tube à l'endroit de la ligne de séparation, dans le cas de la figure 8.

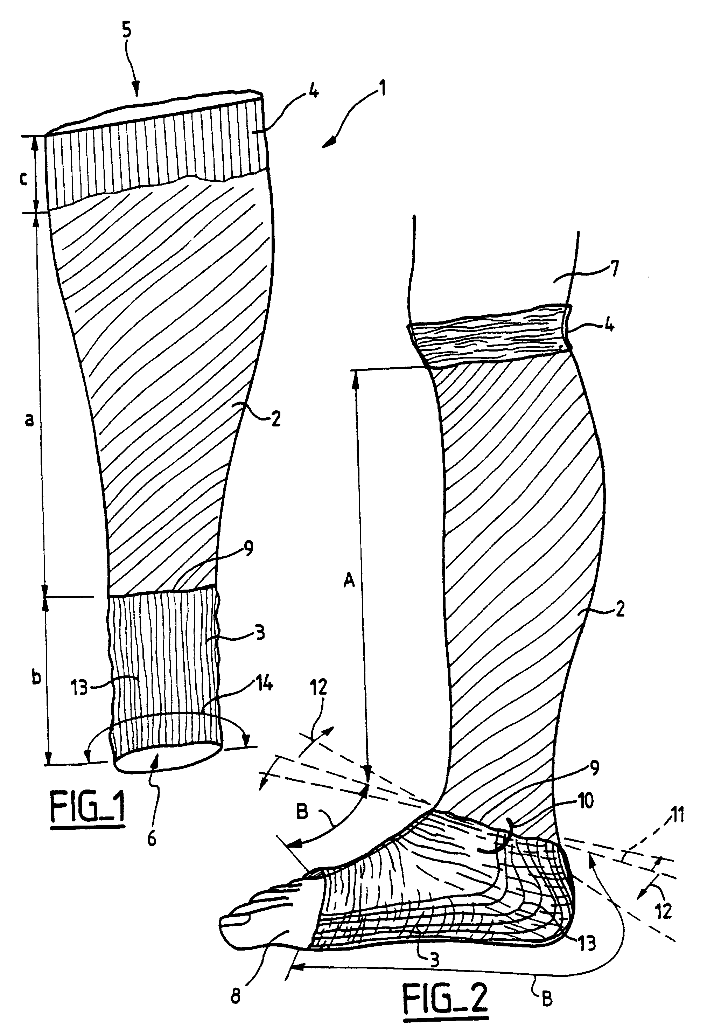

Sur les figures 1 et 2, on a représenté une orthèse de type botte selon l'invention, respectivement à l'état libre (avant enfilage) et en place, après enfilage sur la jambe et le pied d'un patient.

La botte 1 est essentiellement constituée d'une partie centrale 2 en maille compressive dont la forme et la texture correspondent à celles d'un bas de contention de type classique, mais dépourvu de pied et de talon. La longueur a de cette partie compressive variera, d'un article à l'autre, en fonction de la taille de la morphologie du patient, et constituera ensuite un élément compressif appliqué sur le pansement de l'ulcère.

A son extrémité inférieure, distale, cette partie compressive 2 est prolongée par une partie non compressive 3 qui est avantageusement dépourvue de pied et de talon tricotés, c'est-à-dire qu'elle se présente, à l'état libre, comme un simple élément tubulaire ; le caractère suffisamment étirable de sa maille doit permettre d'envelopper facilement le pied sans le comprimer et sans former de pli, comme illustré figure 2.

La longueur b de cette partie non compressive 3 est, à l'état libre, par exemple b = 14 cm, valeur qui permet de recouvrir la majeure partie du pied jusqu'à la racine des orteils, sans pour autant risquer de former de pli indésirable. Cette partie 3 s'étend sur une longueur B variable, faible sur le dessus du pied et beaucoup plus grande sur la face inférieure en raison de la présence du talon qui doit être enveloppé.

En partie supérieure, proximale, la partie compressive 2 peut être prolongée par une autre partie non compressive 4, dont la longueur c peut être assez réduite, par exemple c = 8 cm.

La botte de l'invention se présente ainsi avant enfilage sous forme d'un tube tricoté à section variable, comportant une ouverture supérieure 5 par laquelle la botte sera enfilée et une ouverture inférieure 6 permettant de laisser libre l'extrémité du pied.

Une fois mise en place sur la jambe 7 et le pied 8 du patient, la partie compressive centrale 2 va réaliser une contention de la jambe sur une longueur A incluant la région de l'ulcère avec son pansement.

En particulier, la structure de la botte de l'invention permet un ajustement et un réajustement faciles de la ligne de transition 9 entre la partie compressive 2 et la partie non compressive 3, correspondant à la zone de début de compression (et également de compression maximale).

Le praticien peut ainsi assurer un ajustement précis de la position 11 de la ligne de transition 9, ainsi que l'inclinaison de cette ligne (flèches 12) de manière à bien couvrir tous les cas de figures possibles, tout particulièrement pour les ulcères situés dans la région de la malléole 10, situation assez fréquente. La ligne 9 peut ainsi être placée au-dessous de la malléole (en recouvrant une partie du cou-de-pied), passer sur la malléole, ou se situer au-dessus de cette dernière.

La partie inférieure non compressive 3 enveloppe et protège le pied dans toute la région distale attenante à la zone de contention. Il est important que l'extrémité distale de la botte soit ouverte car cette configuration permet au praticien de relever aisément la partie non compressive 3 pour une inspection visuelle de la plaie, un réajustement des pansements, etc. sans qu'il soit nécessaire de retirer la botte.

Pour compenser l'usure de la partie non compressive 3 dans la région du talon, on prévoit avantageusement un fil de renfort 13, non compressif, ajouté à la maille mousse, par exemple un fil de polyamide assez gros. Avantageusement, le renfort de la maille n'est prévu que sur une fraction 14 de la circonférence du tube formé par la partie non compressive 3, par exemple sur la moitié de cette circonférence, comme illustré en 14 sur la figure 1, qui est une vue de la face postérieure de la botte.

Le dimensionnement des différentes parties est le choix des mailles de tricotage doit permettre de répondre, dans l'indication envisagée, à l'ensemble des critères suivants :
― en ce qui concerne la partie compressive 2 :
   . le degré de compression doit être suffisant pour réaliser la contention voulue, mais avec une pression inférieure à celle qui serait appliquée pour une réduction de lymphoedème (on ne cherche pas à obtenir un drainage, mais à comprimer perpendiculairement à la peau) ;
   . la compression doit être dégressive à partir de la cheville, et effective seulement à partir de la cheville (notamment à la différence d'une réduction de lymphoedème, où cette compression doit être appliquée à partir des orteils) ;
   . le pied ne doit surtout pas être comprimé, de la même façon que pour les bas de contention de type classique où la compression sur le pied est pratiquement nulle, grâce à la présence d'un pied et d'un talon tricotés.
― en ce qui concerne l'élasticité de la maille, la force de rappel élastique doit être compatible avec le port de la botte en décubitus la nuit, lorsque se produit une diminution importante des pressions veineuse et oncotique. Une certaine élasticité doit cependant être présente et adaptée à la morphologie du patient, car la nécessité du port continu, jour et nuit, du produit durant plusieurs jours, voir plusieurs semaines, ne permet pas l'utilisation d'un produit non extensible ni, au contraire, d'un produit qui produirait une force de rappel trop élevée insupportable en décubitus.
― le produit doit être facilement enfilable sur des jambes déformées, aux téguments fragiles, sur lesquels sont appliqués des pansements volumineux recouvrant les plaques d'hydrocolloïdes, traitement aujourd'hui recommandé pour la réduction des ulcères veineux de jambes.
― le réajustement de la botte et des pansements à la cheville doit être possible, grâce notamment à l'absence de pied tricoté et de pression exercée sur le pied.

En ce qui concerne la fabrication de la botte de l'invention, cette botte peut être avantageusement réalisée sous forme d'un "tube" tricoté en continu de bottes individuelles reliées entre elles, découpées après fabrication, ou ultérieurement par le praticien pour les appliquer ensuite sur le pansement de l'ulcère.

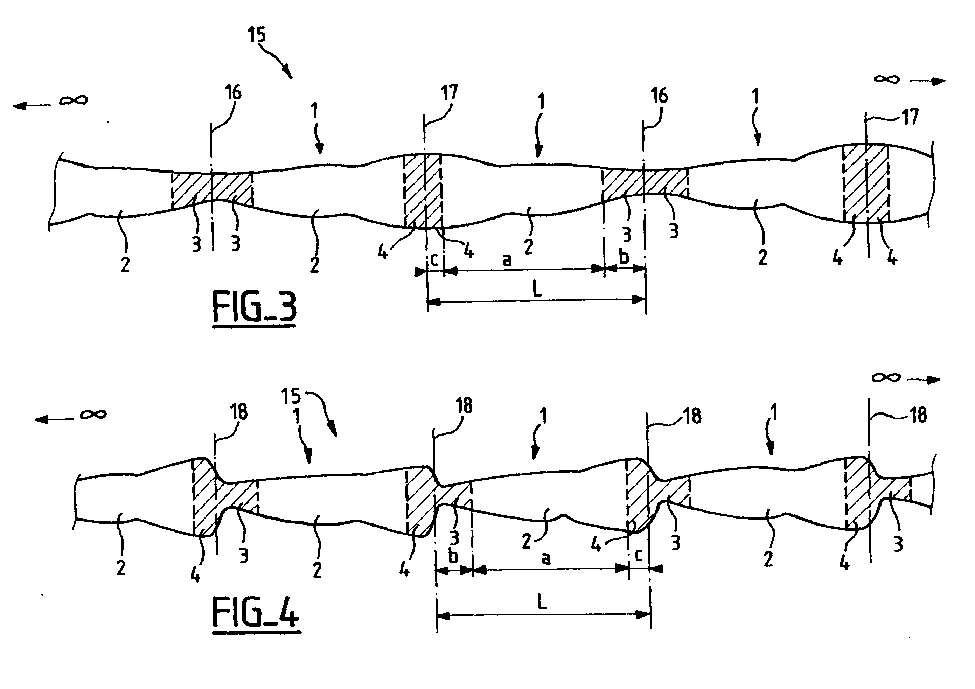

Dans la mise en oeuvre illustrée figure 3, le tube 20 est formé de bottes successives 1, 2 qui ont une configuration symétrique de part et d'autre d'axes 16, 17, respectivement au niveau des chevilles et des cuisses (ou des jarrets), c'est-à-dire que les bottes sont reliés entre elles successivement par les chevilles puis par les cuisses. Chaque élément 1 peut être séparé en un élément individuel de longueur L comprenant la partie de jambe 2 compressive s'étendant entre la région de cuisse 4 non compressive et la région de cheville 3 non compressive.

En variante, comme illustré figure 4, les éléments 1 peuvent être tous disposés dans le même sens, avec à l'endroit de chaque axe 18 une région de cuisse 4 venant se raccorder à une région de cheville 3 de la botte adjacente.

Le tube 15 est tricoté en continu (donc avec une cadence élevée et un faible prix de revient), avec une section variable de façon à épouser la morphologie de la jambe correspondante et assurer la compression dégressive recherchée.

La maille est de type classique, mais doit être indémaillable pour ne pas "filer" aux deux extrémités. Elle est choisie pour délivrer, dans la région de la partie de jambe 2 une compression correspondant à une classe II ou III dégressive, classe de contention convenant en particulier au traitement des ulcères veineux. Dans les parties non compressives 3 et 4, la maille est par exemple de type jersey mousse.

La botte peut être réalisée en fil guipé ou non et ne nécessite aucune teinture après tricotage. Son coût de revient peut ainsi être très bas et donc compatible avec celui d'un produit à usage unique.

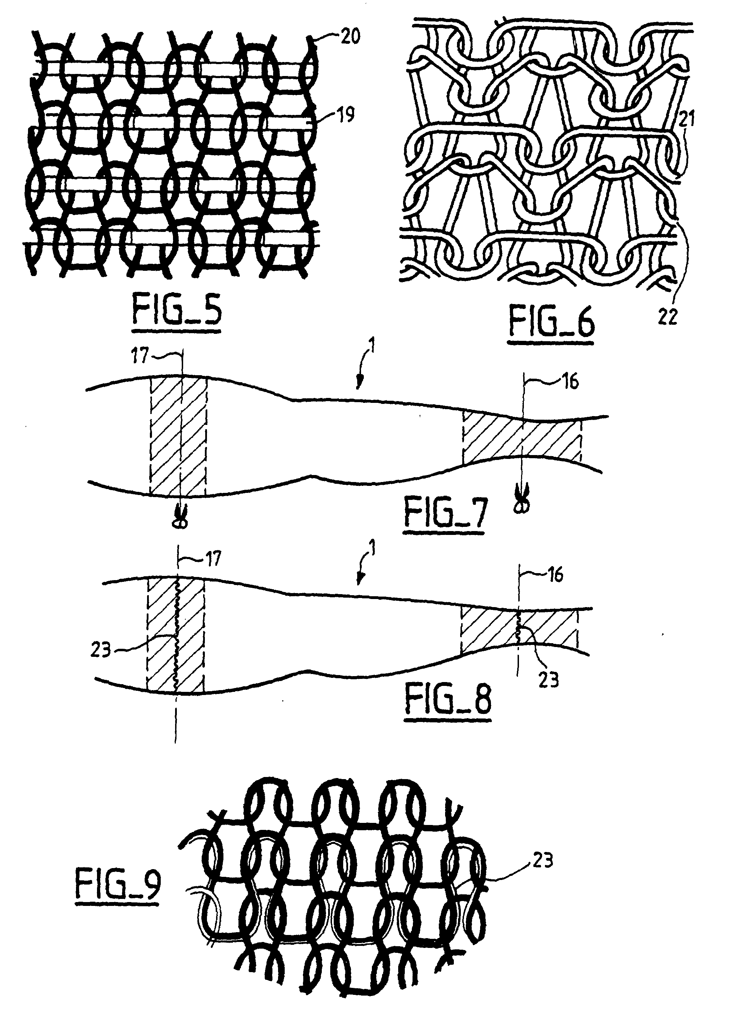

Le tricotage peut être par exemple, comme illustré figure 5, un tricotage circulaire avec une trame : la référence 19 désigne le fil de trame, qui est un fil guipé ou non, élastique dans la partie de jambe 2, et la référence 20 désigne le fil de tricot, élastique ou non. Cette structure est une structure classique de bas de contention.

En variante, on peut adopter pour la partie de jambe 2 la structure du type bas de maintien ou de soutien illustrée figure 6, par exemple une structure du type micromesh 1 x 1 avec un fil élastique 21 texturé ou plat et un fil élastique 22 guipé ou non, ou encore une structure micromesh 1 x 1 flottée alternée, unie, etc.

Quelle que soit la structure choisie, les fils non élastiques peuvent être par exemple des fils du type polyamide plats ou texturés, et les fils élastiques des fils guipés (guipage traditionnel ou guipage *Air Jet* par exemple) ou nus (élasthanne, latex naturel, etc.).

Pour le découpage du tube en bottes individuelles, dans une première variante illustrée figure 7, on se contente d'une découpe avec des ciseaux dans la région de cuisse 4 et la région de cheville 3, éventuellement avec ajustement de la longueur.

En variante, comme illustré figure 8, on peut prévoir au niveau des zones de séparation un fil sécable spécial 23 (type *EMS* ou *Luxilon* par exemple) ou thermofusible, pour permettre une séparation plus aisée des différentes bottes. La figure 9 montre la présence de ce fil sécable 23 dans la maille au niveau de la zone de découpe, ce fil pouvant être prévu transversalement par exemple sur une ou deux rangées du tube.

## Revendications

1. Une orthèse compressive de type botte pour la contention de la jambe à la suite d'un ulcère veineux, cette orthèse étant apte à appliquer une compression dégressive à partir de la cheville sur tout ou partie de la jambe,
cette orthèse (1) comportant une partie tubulaire compressive (2) tricotée à section variable, formée par une partie de jambe d'un bas de contention, apte à réaliser une contention de la jambe sur une longueur incluant la région de l'ulcère avec son pansement,
orthèse **caractérisée en ce que**
- ladite partie de jambe de bas de contention est dépourvue de pied et de talon,
- ladite partie tubulaire compressive est prolongée à son extrémité inférieure par une partie non compressive (3) tricotée, dépourvue de pied et de talon tricotés, se présentant à l'état libre comme un élément tubulaire ouvert, et
- la maille de cette partie non compressive tubulaire tricotée est une maille étirable choisie de manière à pouvoir recouvrir, sans le comprimer et sans former de pli, au moins une partie du pied jusqu'à la racine des orteils.

2. L'orthèse de type botte de la revendication 1, dans laquelle la partie tubulaire non compressive comporte dans sa maille des fils de renfort (13) sur au moins une partie de sa circonférence.

3. L'orthèse de type botte de la revendication 1, dans laquelle la partie tubulaire non compressive est réalisée en une maille de type jersey mousse.

4. L'orthèse de type botte de la revendication 1, dans laquelle la partie tubulaire compressive est prolongée à son extrémité supérieure par une autre partie tubulaire non compressive (4) tricotée.

5. L'orthèse de type botte de la revendication 1, **caractérisée en ce qu'**elle est réalisée sous forme d'un tube tricoté en continu à section variable formé d'une maille indémaillable alternativement compressive et non compressive, ce tube étant constitué d'une suite d'éléments individuels (1) semblables séparables par découpe du tube, ces éléments formant chacun une botte individuelle s'étendant entre deux zones de découpe délimitant la botte et situées dans les régions de maille non compressive.

6. L'orthèse de type botte de la revendication 5, dans laquelle les éléments individuels forment une suite d'éléments disposés en opposition, chaque élément étant déduit de l'élément adjacent par symétrie de part et d'autre d'axes (16, 17) situés dans la région des zones de découpe.

7. L'orthèse de type botte de la revendication 5, dans laquelle les éléments individuels forment une suite d'éléments disposés en séquence, chaque élément étant déduit de l'élément adjacent par une translation d'un module égal à la longueur d'un élément individuel.

8. L'orthèse de type botte de la revendication 5, dans laquelle la maille du tube comporte au moins un fil sécable (23) dans chaque zone de découpe.

## Patentansprüche

1. Eine Kompressionsorthese vom Typ eines Stiefels für das Fixieren des Beines nach einem venösen Geschwür, wobei diese Orthese angepaßt ist, eine abnehmende Kompression ausgehend vom Knöchel über das gesamte oder einen Teil des Beines auszuüben,
wobei diese Orthese einen röhrenförmigen komprimierenden Teil (2) aufweist, der gestrickt bzw. gewirkt ist mit variablem Abschnitt, der durch einen Teil des Beines eines Fixierstrumpfs gebildet ist, geeignet, um ein Fixieren des Beines über eine Länge einschließlich des Bereichs des Geschwürs mit seinem Verband herzustellen, wobei die Orthese **dadurch gekennzeichnet** ist, daß
- der Teil des Beines des Fixierstrumpfs ohne Fuß und ohne Ferse ist,
- der komprimierende röhrenförmige Teil an seinem unteren Ende durch einen nicht komprimierenden gewirkten Teil (3) verlängert ist, ohne gewirkten Fuß und Ferse, sich im freien Zustand wie ein offenes röhrenförmiges Element darstellend, und
- das Maschenwerk dieses nicht komprimierenden röhrenförmigen gewirkt ten Teils ein streckbares Maschenwerk ist, das so gewählt ist, daß es zumindest einen Teil des Fußes bis zur Wurzel der Zehen überdecken kann, ohne ihn zu komprimieren und ohne Bilden einer Falte.

2. Orthese vom Typ eines Stiefels nach Anspruch 1, bei welcher der röhrenförmige nicht komprimierende Teil in seinem Maschenwerk Verstärkungsfäden, (13) über zumindest einen Teil seines Umfangs ausweist.

3. Orthese vom Typ eines Stiefels nach Anspruch 1, bei welcher der röhrenförmige nicht komprimierende Teil aus einem Maschenwerk des Typs eines Jersey-Schaums hergestellt ist.

4. Orthese vom Typ eines Stiefels nach Anspruch 1, bei welcher der röhrenförmige komprimierende Teil an seinem oberen Ende durch einen weiteren nicht komprimierenden gewirkten Teil (4) verlängert ist.

5. Orthese vom Typ eines Stiefels nach Anspruch 1, **dadurch gekennzeichnet, daß** sie in Form eines durchgehend gewirkten Tubus mit variablem Abschnitt hergestellt ist, gebildet aus einem laufmaschenfesten Maschenwerk, abwechselnd komprimierend und nicht komprimierend, wobei dieser Tubus aus einer Folge gleichartiger individueller Elemente (1) gebildet ist, die durch Zerschneiden des Tubus trennbar sind, wobei diese Elemente jeweils einen individuellen Stiefel formen, der sich zwischen zwei Schneidezonen erstreckt, die den Stiefel abgrenzen und sich in nicht komprimierenden Maschenbereichen befinden.

6. Orthese vom Typ eines Stiefels nach Anspruch 5, bei welcher die individuellen Elemente eine Folge von Elementen bilden, die gegenüberliegend angeordnet sind, wobei jedes Element vom benachbarten Element durch Symmetrie beiderseits von Achsen (16, 17) hergeleitet ist, die sich in dem Bereich der Schneidezonen befinden.

7. Orthese vom Typ eines Stiefels nach Anspruch 5, bei welcher die individuellen Elemente eine Folge von Elementen bilden, die der Reihe nach angeordnet sind, wobei jedes Element vom benachbarten Element durch eine Translation eines Moduls hergeleitet ist, das gleich der Länge eines individuellen Elementes ist.

8. Orthese vom Typ eines Stiefels nach Anspruch 5, bei welcher das Maschenwerk des Tubus zumindest einen teilbaren Faden (23) in jeder Schneidezone aufweist.

## Claims

1. A compressive orthosis of the sock type for compressive support of the leg after a venous ulcer, said orthosis being suitable for applying over all or part of the leg a compressive support which is degressive from the ankle,
said orthosis (1) including a knitted compressive tubular portion (2) of varying section constituted by a leg portion of an elastic stocking suitable for applying compressive support of the leg upon a length thereof including the region of said ulcer along with its dressing,
said orthosis being **characterized in that** :
- said leg portion of an elastic stocking has no foot or heel,
- said compressive tubular portion is extended at its bottom end by a knitted non-compressive tubular portion (3), which has no knitted foot or heel and which constitutes in the free state an open tubular element, and
- the mesh of said knitted non-compressive tubular portion is a stretchable mesh which is selected so as to cover at least part of the foot up to the base of the toes, without compressing it and without forming creases.

2. The sock type orthosis of claim 1, in which the non-compressive tubular portion has reinforcing yarns (13) in its knitting, over at least a portion of its circumference.

3. The sock type orthosis of claim 1, in which the non-compressive tubular portion is made using a plain moss type stitch.

4. The sock type orthosis of claim 1, in which the compressive tubular portion is extended at its top end by another knitted non compressive tubular portion (4).

5. The sock type orthosis of claim 1, **characterized in that** it is made in the form of an continuous knitted tube of varying section made of non-run stitch that is alternately compressive and non-compressive, the tube being constituted by a sequence of similar individual elements (1) separable by cutting the tube, each of said elements forming an individual sock extending between two cutting-out zones defining the sock and situated in the regions of non-compressive knitting.

6. The sock type orthosis of claim 5, in which the individual elements form a sequence disposed in opposite directions, each element being the reflection of an adjacent element about axes of symmetry (16, 17) situated in the regions of the cutting-out zones.

7. The sock type orthosis of claim 5, in which the individual elements form a sequence of elements disposed the same way round, each element being a copy of an adjacent element representing a shift of a module of length equal to one individual element.

8. the sock type orthosis of claim 5 , in which the knitting of the tube includes at least one breakable thread (23) in each cutting-out zone.
